# FASCICULE DE BREVET EUROPEEN

(11) **EP 0 904 777 B1**
(45) Date de publication et mention de la délivrance du brevet: **26.11.2008**
(21) Numéro de dépôt: 98402327.5
(22) Date de dépôt: 22.09.1998
(51) Int. Cl.: A61K 8/64, A61Q 19/00

(54) **Utilisation d'un inhibiteur d'acides aminés excitateurs dans une composition cosmétique ou dermatologique pour peaux sensibles et composition obtenue**
Verwendung von Inhibitoren exzitatorischer Aminosäuren in kosmetischen oder dermatologischen Zubereitungen für empfindliche Haut sowie Zubereitung
Use of an inhibitor of excitatory amino acids in cosmetic or dermatological compositions for sensitive skin and composition obtained

(30) Priorité: 25.09.1997 FR 9711959
(43) Date de publication de la demande: 31.03.1999
(73) Titulaire: L'ORÉAL, 75008 Paris (FR)
(72) Inventeur: Breton, Lionel, 78000 Versailles (FR); Nonotte, Isabelle, 75017 Paris (FR)
(74) Mandataire: Bernstein, Claire Jacqueline

(56) Documents cités:
- EP-A- 0 529 636
- EP-A- 0 680 749
- EP-A- 0 717 997
- WO-A-92/17168
- WO-A-97/10815
- D. H. SMULLIN ET AL: "Interactions between substance P, calcitonin gene-related peptide, taurine and excitatory amino acids in the spinal cord" PAIN, vol. 42, 1990, pages 93-101, XP000675202

## Description

La présente invention se rapporte à l'utilisation d'un inhibiteur d'au moins un acide aminé excitateur, tel que l'aspartate et le glutamate, pour la préparation d'une composition dermatologique, destinée à traiter les peaux sensibles, y compris le cuir chevelu, les contours des yeux et les muqueuses, d'êtres humains. Elle se rapporte aussi à la composition dermatologique obtenue.

Il est connu que certaines peaux sont plus sensibles que d'autres. Or, les symptômes des peaux sensibles étaient jusqu'à présent mal caractérisés et le problème de ces peaux était, de ce fait, mal défini ; personne ne connaissait exactement le processus mis en cause dans la sensibilité de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques.

Certains tests ont été essayés pour tenter de cerner les peaux sensibles, par exemple des tests à l'acide lactique et au DMSO qui sont connus pour être des substances irritantes : voir par exemple l'article de K. Lammintausta et al., Dermatoses, 1988, 36, pages 45-49; et l'article de T. Agner et J. Serup, Clinical and Experimental Dermatology, 1989, 14, pages 214-217. Mais ces tests ne permettaient pas de caractériser les peaux sensibles.

Par ailleurs, on assimilait les peaux sensibles à des peaux allergiques.

Du fait que l'on connaissait mal les caractéristiques des peaux sensibles, il était jusqu'à présent très difficile de les traiter, et on les traitait indirectement, par exemple en limitant dans la composition cosmétique l'emploi de produits à caractère irritant tels que les tensioactifs, les conservateurs, les parfums ainsi que certains actifs.

La demanderesse a réalisé de nombreux tests cliniques et a su déterminer les symptômes liés aux peaux sensibles. Ces symptômes sont en particulier des signes subjectifs, qui sont essentiellement des sensations dysesthésiques. On entend par « sensations dysesthésiques » des sensations plus ou moins douloureuses ressenties dans une zone cutanée comme les picotements, fourmillements, démangeaisons ou prurits, brûlures, échauffements, inconforts, tiraillements, etc.

Par ailleurs, la demanderesse a pu montrer qu'une peau sensible n'était pas une peau allergique. La caractéristique essentielle de la peau sensible est selon la demanderesse, un mécanisme de réponse à des facteurs extérieurs, qui peut concerner tout individu, même si les individus dits à peau sensible y réagissent plus vite que les autres. Ce mécanisme n'est pas immunologique.

La demanderesse a maintenant trouvé que les peaux sensibles pouvaient être scindées en deux grandes formes cliniques, les peaux irritables et/ou réactives, et les peaux intolérantes.

Une peau irritable et/ou réactive est une peau qui réagit par un prurit, c'est-à-dire par des démangeaisons ou par des picotements, à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche ou à une peau qui présente un érythème.

Une peau intolérante est une peau qui réagit par des sensations d'échauffement, de tiraillements, de fourmillements et/ou de rougeurs, à différents facteurs tels que l'environnement, les émotions, les aliments. En général, ces signes sont associés à une peau hyperséborrhéique ou acnéique et à un érythème.

Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs, eau dure à forte concentration de calcaire, shampooings ou lotions, produits de mise en forme permanente. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à l'eau dure à forte concentration en calcaire et/ou aux variations de température.

Pour déterminer si une peau est sensible ou non, la demanderesse a également mis au point un test. En effet, après avoir effectué un grand nombre de tests dans le but de définir une peau sensible, elle a trouvé de manière surprenante qu'il existait un lien entre les personnes à peau sensible et celles qui réagissaient à une application topique de capsaïcine.

Le test à la capsaïcine consiste à appliquer sur environ 4 cm² de peau 0,05 ml d'une crème contenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

La capsaïcine provoque notamment une libération des neuropeptides, et en particulier des tachykinines qui proviennent de terminaisons nerveuses sensitives de l'épiderme et du derme. La demanderesse a constaté que le schéma physiopathologique commun à tous les états des peaux sensibles était lié à une grande aptitude à libérer des tachykinines et plus particulièrement de la substance P dans la peau. Les manifestations dysesthésiques qui sont provoquées par la libération de neuropeptides sont dites "neurogènes".

Cette substance P est un polypeptide élaboré et libéré par les terminaisons nerveuses sensitives. Elle induit notamment une dégranulation des mastocytes entraînant la libération de sérotonine ainsi qu'une cascade d'événements biochimiques aboutissant à une réaction inflammatoire. La libération de substance P par les terminaisons nerveuses sensitives peut être contrôlée par différents agents dont les acides aminés excitateurs tels que le glutamate et/ou l'aspartate.

La demanderesse a maintenant découvert que des fibres nerveuses présentes dans l'épiderme et/ou le derme contenaient des acides aminés excitateurs tel que l'aspartate et/ou le glutamate. Ainsi, la libération de l'aspartate et/ou du glutamate à proximité des terminaisons nerveuses sensitives active la libération de substance P et donc la cascade d'événements biochimiques qui engendre des réactions nociceptives plus ou moins importantes. Par conséquent, l'utilisation d'un inhibiteur d'au moins un acide aminé excitateur permet d'obtenir un effet préventif et/ou curatif sur les peaux sensibles. En effet, en diminuant la libération et/ou la synthèse d'acides aminés excitateurs par les fibres nerveuses et/ou en diminuant leur fixation, l'activité observée de ces acides aminés excitateurs est diminuée.

Pour traiter les peaux sensibles, la demanderesse a donc envisagé d'utiliser un inhibiteur d'au moins un acide aminé excitateur. Elle a en effet constaté de manière surprenante que l'incorporation d'un inhibiteur d'au moins un acide aminé excitateur dans une composition dermatologique permettait d'éviter l'irritation et/ou les sensations dysesthésiques et/ou les prurits de la peau.

La présente demande décrit l'utilisation d'au moins un inhibiteur d'au moins un acide aminé excitateur pour la préparation d'une composition dermatologique, contenant un milieu physiologiquement acceptable, pour traiter les peaux sensibles.

La présente invention a pour objet l'utilisation d'au moins un inhibiteur d'au moins un acide aminé excitateur pour la préparation d'une composition dermatologique, contenant un milieu physiologiquement acceptable, pour prévenir et/ou lutter contre les irritations cutanées et/ou les dartres et/ou les érythèmes et/ou les prurits de la peau.

De façon plus générale, l'invention se rapporte à l'utilisation d'au moins un inhibiteur d'au moins un acide aminé excitateur pour la préparation d'une composition dermatologique, contenant un milieu physiologiquement acceptable, pour traiter les symptômes cutanés liés à la libération et/ou la synthèse et/ou la fixation d'au moins un acide aminé excitateur.

Un milieu physiologiquement acceptable est un milieu cosmétiquement ou dermatologiquement acceptable, qui est compatible avec la peau, les muqueuses, les ongles et les cheveux. En particulier, la composition contenant un inhibiteur d'au moins un acide aminé excitateur peut être appliquée sur le visage, le cou, les cheveux et les ongles, ou toute autre zone cutanée du corps.

Pour qu'une substance soit reconnue comme un inhibiteur d'au moins un acide aminé excitateur, elle doit inhiber la libération et/ou la fixation et/ou la synthèse d'au moins un acide aminé excitateur, tel que le glutamate et/ou l'aspartate, au niveau du derme et/ou de l'épiderme. Elle doit, plus particulièrement, répondre au moins à l'une des caractéristiques suivantes :
- avoir une activité pharmacologique, mesurée notamment par électrophorèse, de type antagoniste réceptoriel à l'un des récepteurs d'au moins un acide aminé excitateur, tel que notamment les récepteurs de type AMPA (L-α-amino-3-hydroxy-5-méthyl-4-isoxazole propionate), de type NMDA (N-methyl-D-aspartate) ou de type kainate (la pharmacologie et la localisation permettent de différencier ces récepteurs qui sont tous trois couplés à un canal ionique) ;
- diminuer l'extravasation plasmatique, mesurée notamment par la méthode du Bleu Evans, observée après stimulation antidromique du nerf saphène ou après stimulation à la capsaïcine ;
- diminuer la cytotoxicité cellulaire induite sur des lignées cellulaires neuronales après incubation avec des concentrations élevées de glutamate.

Les signes cliniques de la peau sensible sont essentiellement subjectifs, à savoir : picotements, fourmillements, démangeaisons ou prurits, tiraillements, échauffements ; ils s'accompagnent parfois d'érythèmes. Ces signes sont dus à des facteurs extérieurs aspécifiques.

Selon l'invention on peut utiliser un ou plusieurs inhibiteurs d'au moins un acide aminé excitateur.

On peut ainsi utiliser comme inhibiteur d'au moins un acide aminé excitateur des molécules de synthèse chimique et également des extraits d'origine animal, végétal ou bactérien.

Parmi les inhibiteurs d'au moins un acide aminé excitateur, on peut notamment citer la kétamine, la memantine, (±)-2-amino-3-phosphonopropionic acid, (±)-2-amino-4-phosphonobutyric acid, (±)-2-amino-5-phosphonopentanoic acid, l'acide D-8-Glutamylaminométhane sulfonique, le 1,2,3,6,7,8-hexahydro-3-(hydroxyimino)-N,N,7-trimethyl-2-oxo-benzo[2,1-b:3,4-c']dipyrrole-5 sulfonamide hydrochloride (appelé de façon plus commune: NS 257, HCl), la Cyclothiazide, l'Aniracetam et le riluzole.

De préférence, l'inhibiteur d'au moins un acide aminé excitateur est choisi parmi l'aniracetam et la kétamine.

La quantité d'inhibiteur d'acide aminé excitateur dans une composition varie dans une large mesure, notamment en fonction du pouvoir inhibiteur du composé et de l'effet recherché.

Pour donner un ordre de grandeur, dans la composition selon l'invention, l'inhibiteur d'au moins un acide aminé excitateur est utilisé de préférence en une quantité allant de 0,000001 à 5 % du poids total de la composition, et en particulier en une quantité allant de 0,0001 à 1 %.

La composition selon l'invention peut se présenter sous toute forme galénique normalement utilisée pour une application topique sur la peau, notamment sous forme de solution ou dispersion du type lotion ou sérum, d'émulsion de consistance liquide ou semi-liquide du type lait, obtenue par dispersion d'une phase grasse dans une phase aqueuse (H/E) ou inversement (E/H), ou de suspension ou émulsion de consistance molle du type crème ou gel aqueux ou anhydre, de microgranulés, de nanoparticules, de microémulsion, de nanocapsules ou de dispersions de vésicules de type ionique et/ou non ionique. Ces compositions sont préparées selon les méthodes usuelles.

Elle peut également être utilisée pour les cheveux sous forme de solution aqueuse, alcoolique ou hydroalcoolique, de crème, de gel, d'émulsion, de mousse ou encore sous forme de compositions pour aérosol contenant un agent propulseur sous pression.

Les quantités des différents constituants de la composition selon l'invention sont celles classiquement utilisées dans les domaines considérés.

La composition de l'invention constitue notamment une crème de nettoyage, de protection, de traitement ou de soin pour le visage, pour les mains, pour les pieds, pour les grands plis anatomiques ou pour le corps, (par exemple crème de jour, crème de nuit, crème démaquillante, crème de fond de teint, crème anti-solaire), un fond de teint fluide, un lait de démaquillage, un lait corporel de protection ou de soin, un lait anti-solaire, une lotion, un gel ou une mousse pour le soin de la peau, comme une lotion de nettoyage, une lotion anti-solaire, une lotion de bronzage artificiel, une composition pour le bain, une composition désodorisante contenant un agent bactéricide, un gel ou lotion après-rasage, une crème épilatoire, une composition contre les piqûres d'insectes, une composition anti-douleur.

La composition selon l'invention peut également consister en une préparation solide constituant un savon ou un pain de nettoyage.

L'inhibiteur d'au moins un acide aminé excitateur peut aussi être incorporé dans tout type de composition pour soins capillaires, et notamment dans un shampooing, une lotion de mise en plis, une lotion traitante, une crème ou gel coiffant, une composition de teinture (notamment teinture d'oxydation) éventuellement sous forme de shampooing colorant, une lotion restructurante pour les cheveux, une composition de permanente (notamment une composition pour le premier temps d'une permanente), une lotion ou gel antichute, etc.

La composition de l'invention peut aussi être à usage bucco-dentaire, par exemple sous forme de pâte dentifrice. Dans ce cas, la composition peut contenir des adjuvants et additifs usuels pour une composition à usage buccal et notamment des agents tensioactifs, épaississants ou humectants, des agents de polissage tels que la silice, ou divers ingrédients actifs comme les fluorures, en particulier le fluorure de sodium, et éventuellement des agents édulcorants comme le saccharinate de sodium.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants alors employés sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 à 30 % en poids par rapport au poids total de la composition. L'émulsion peut, en outre, contenir des vésicules lipidiques.

De façon connue, la composition de l'invention peut également contenir des adjuvants habituels dans le domaine considéré, tels que les gélifiants hydrophiles ou lipophiles, les actifs hydrophiles ou lipophiles, les conservateurs, les antioxydants, les solvants, les parfums, les charges, les filtres et les matières colorantes. Les quantités de ces différents adjuvants sont celles classiquement utilisées dans le domaine considéré, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{®} 63 par la société Gattefosse.

Comme solvants utilisables dans l'invention, on peut citer les alcools inférieurs, notamment l'éthanol et l'isopropanol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe.

Comme actifs hydrophiles, on peut utiliser les protéines ou les hydrolysats de protéine, les acides aminés, les polyols, l'urée, l'allantoïne, les sucres et les dérivés de sucre, les vitamines et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

On peut entre autres associer l'inhibiteur d'au moins un acide aminé excitateur à des agents actifs destinés notamment à la prévention et/ou au traitement des affections cutanées.

Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine,
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, ou la triméprazine;
- les agents antiviraux tels que l'acyclovir ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et les dérivés alkylés en C₂-C₂₂ de l'acide salicylique comme l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

La présente demande décrit un procédé de traitement dermatologique, caractérisé en ce qu'on applique sur la peau, les cheveux, et/ou les muqueuses, plus particulièrement d'êtres humains, une composition telle que décrite ci-dessus contenant au moins un inhibiteur d'au moins un acide aminé excitateur dans un milieu dermatologiquement acceptable.

Plus particulièrement, la composition est appliquée sur les peaux sensibles et/ou les cuirs chevelus sensibles.

On a vu précédemment dans le texte ce qu'est une peau irritable. L'irritation cutanée peut avoir de multiples causes. Ce peut être des causes intrinsèques, liés au dérèglement des mécanismes physiologiques conduisant à une peau normale. Mais ce peut être également des causes extrinsèques comme des composés irritants qui viendraient en contact avec la peau.

Ainsi, le traitement est destiné à diminuer l'irritation cutanée.

Ce procédé de traitement peut être mis en oeuvre notamment en appliquant la composition définie ci-dessus, selon la technique d'utilisation habituelle de ce type de composition. Par exemple : application sur la peau ou les muqueuses de crème, de gel, de sérum, de lotion, de lait de démaquillage, de composition anti-solaire ou sur les cheveux secs ou mouillés, de shampooings ou après shampooings, ou encore application sur les gencives de dentifrice.

Aussi, de façon avantageuse, l'inhibiteur d'au moins un acide aminé excitateur est associé à des actifs ayant un effet secondaire irritant, utilisés couramment dans les domaines considérés. La présence de cet inhibiteur d'au moins un acide aminé excitateur dans une composition de l'invention contenant un actif à effet irritant permet d'atténuer fortement, voire de supprimer cet effet irritant.

Aussi, l'invention a encore pour objet une composition cosmétique ou dermatologique contenant un milieu physiologiquement acceptable et au moins un actif à effet secondaire irritant, caractérisée en ce qu'elle contient, en outre, au moins un inhibiteur d'au moins un acide aminé excitateur.

En particulier, les actifs à effet secondaire irritant sont choisis parmi les α-hydroxy-acides (acide de fruits), les β-hydroxy-acides comme l'acide salicylique et ses dérivés alkylés, les α-céto-acides, les β-céto-acides, les rétinoïdes, les anthralines, les anthranoïdes, les peroxydes, les sels de lithium, les antimétabolites, la vitamine D et ses dérivés.

Les exemples suivants sont donnés à titre illustratif. Dans ces exemples, les proportions sont données en pourcentages en poids.

### Exemple 1 : lotion démaquillante pour le visage

| | |
|---|---|
| Aniracetam | 0,0001 |
| Antioxydant | 0,05 |
| Isopropanol | 40,00 |
| Conservateur | 0,30 |
| Eau | qsp 100 % |

### Exemple 2 : Gel pour le soin du visage

| | |
|---|---|
| Aniracetam | 0,05 |
| Hydroxypropylcellulose (Klucel H vendu par la société Hercules) | 1,00 |
| Antioxydant | 0,05 |
| Isopropanol | 30,00 |
| Conservateur | 0,30 |
| Eau | qsp 100 % |

### Exemple 3 : Crème de soin dermatologique du visage (émulsion huile dans eau)

| | |
|---|---|
| Kétamine | 0,002 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

### Exemple 4 : Crème de soin antirides pour le visage (émulsion huile-dans-eau)

| | |
|---|---|
| Aniracetam | 0,15 |
| Stéarate de glycérol | 2,00 |
| Polysorbate 60 (Tween 60 vendu par la société ICI) | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

### Exemple 5 : Shampooing

| | |
|---|---|
| Aniracetam | 0,0001 |
| Lauryl éther sulfate de sodium et magnésium à 4 moles d'oxyde d'éthylène, vendu sous le nom de TEXAPON ASV par HENKEL (tensioactif anionique) | 6,5 g |
| Hydroxypropylcellulose (Klucel H vendu par la société Hercules) | 1,00 |
| Parfum | 0,50 |
| Conservateur | 0,30 |
| Eau | qsp 100 % |

### Exemple 6 : Gel émulsionné de soin contre les piqûres d'insectes (émulsion huile dans eau)

| | |
|---|---|
| Memantine | 0,01 |
| Huile de Purcellin (vendue par la Société Dragocco) | 7,00 |
| PEG-6/PEG-32/Glycol Stéarate (Tefose^{R} 63 de Gattefosse) | 0,30 |
| Sumatriptan | 0,02 |
| Conservateur | 0,30 |
| Parfum | 0,40 |
| Carbomer | 0,60 |
| Crotamiton | 5,00 |
| Acide glycyrrhétinique | 2,00 |
| Alcool éthylique | 5,00 |
| Triéthanolamine | 0,20 |
| Eau | qsp 100 % |

### Exemple 7 : Crème de soin dermatologique de la rosacée pour le visage (émulsion huile-dans-eau)

| | |
|---|---|
| Ketamine | 0,025 |
| Stéarate de glycérol | 2,00 |
| Polysorbate 60 (Tween 60 vendu par la société ICI) | 1,00 |
| Acide stéarique | 1,40 |
| Métronidazole | 1,00 |
| Triéthanolamine | 0,70 |
| Carbomer | 0,40 |
| Fraction liquide du beurre de karité | 12,00 |
| Huile de vaseline | 12,00 |
| Antioxydant | 0,05 |
| Parfum | 0,5 |
| Conservateur | 0,30 |
| Eau | qsp 100 % |

### Exemple 8 : Crème de soin des peaux sensibles contre l'érythème solaire (émulsion huile-dans-eau)

| | |
|---|---|
| Aniracetam | 0,025 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,5 |
| Conservateur | 0,30 |
| Eau | qsp 100 % |

## Revendications

1. Utilisation d'au moins un inhibiteur d'au moins un acide aminé excitateur pour la préparation d'une composition dermatologique, contenant un milieu physiologiquement acceptable, destinée à prévenir et/ou lutter contre les irritations cutanées et/ou les dartres et/ou les érythèmes et/ou les prurits de la peau.

2. Utilisation selon la revendication précédente, **caractérisée en ce que** un inhibiteur d'au moins un acide aminé excitateur est choisi parmi la kétamine, la memantine, (±)-2-amino-3-phosphonopropionic acid, (±)-2-amino-4-phosphonobutyric acid, (±)-2-amino-5-phosphonopentanoic acid, l'acide D-8-Glutamylaminométhane sulfonique, le 1,2,3,6,7,8-hexahydro-3-(hydroxyimino)-N,N,7-trimethyl-2-oxo-benzo[2,1-b:3,4-c']dipyrrole-5 sulfonamide hydrochloride (appelé de façon plus commune: NS 257, HCl), la Cyclothiazide, l'Aniracetam et le riluzole.

3. Utilisation selon la revendication 2, **caractérisée en ce que** l'inhibiteur d'au moins un acide aminé excitateur est choisi parmi l'aniracetam et la kétamine.

4. Utilisation selon l'une des revendications précédentes, **caractérisée en ce que** l'inhibiteur d'au moins un acide aminé excitateur est utilisé en une quantité allant de 0,000001 à 5 % en poids par rapport au poids total de la composition.

5. Utilisation selon l'une des revendications précédentes, **caractérisée en ce que** l'inhibiteur d'au moins un acide aminé excitateur est utilisé en une quantité allant de 0,0001 à 1 % en poids par rapport au poids total de la composition.

6. Utilisation selon l'une des revendications précédentes, **caractérisée en ce que** le milieu est une solution aqueuse ou hydroalcoolique, une émulsion eau-dans-huile, une émulsion huile-dans-eau, une microémulsion, un gel aqueux, un gel anhydre, un sérum, une dispersion de vésicules.

7. Utilisation selon l'une quelconque des revendications précédentes, **caractérisée en ce que** la composition contient au moins un agent choisi parmi les agents antibactériens, antiparasitaires, antifongiques, anti-inflammatoires, antiprurigineux, anesthésiques, antiviraux, kératolytiques, anti-radicaux libres, antiséborrhéiques, antipelliculaires, antiacnéiques et/ou les agents modulant la différenciation et/ou la prolifération et/ou la pigmentation cutanée.

8. Composition cosmétique ou dermatologique contenant un milieu physiologiquement acceptable et au moins un actif à effet secondaire irritant, **caractérisée en ce qu'**elle contient, en outre, au moins un inhibiteur d'au moins un acide aminé excitateur.

9. Composition selon la revendication 8, **caractérisée en ce que** l'inhibiteur d'au moins un acide aminé excitateur est choisi parmi la kétamine, la memantine, (±)-2-amino-3-phosphonopropionic acid, (±)-2-amino-4-phosphonobutyric acid, (±)-2-amino-5-phosphonopentanoic acid, l'acide D-8-Glutamylaminométhane sulfonique, le 1,2,3,6,7,8-hexahydro-3-(hydroxyimino)-N,N,7-trimethyl-2-oxo-benzo[2,1-b:3,4-c']dipyrrole-5 sulfonamide hydrochloride (appelé de façon plus commune: NS 257, HCI), la Cyclothiazide, l'Aniracetam et le riluzole.

10. Composition selon l'une des revendications 8 ou 9, **caractérisée en ce que** l'inhibiteur d'au moins un acide aminé excitateur est utilisé en une quantité allant de 0,000001 à 5 % du poids total de la composition.

11. Composition selon l'une des revendications 8 à 10, **caractérisée en ce que** l'inhibiteur d'au moins un acide aminé excitateur est utilisé en une quantité allant de 0,0001 à 1 % du poids total de la composition.

12. Composition selon l'une des revendications 8 à 11, **caractérisée en ce que** l'actif à effet secondaire irritant est choisi parmi les α-hydroxy-acides, les β-hydroxy-acides, les α-céto-acides, les β-céto-acides, les rétinoïdes, les anthralines, les anthranoïdes, les peroxydes, les sels de lithium, les antimétabolites, la vitamine D et ses dérivés.

13. Composition selon l'une des revendications 8 à 12, **caractérisée en ce qu'**elle contient, en outre, au moins un agent choisi parmi les agents antibactériens, antiparasitaires, antifongiques, anti-inflammatoires, antiprurigineux, anesthésiques, antiviraux, kératolytiques, antiradicaux libres, anti-séborrhéiques, antipelliculaires, antiacnéiques et/ou les agents modulant la différenciation et/ou la prolifération et/ou la pigmentation cutanée.

## Claims

1. Use of at least one inhibitor of at least one excitatory amino acid for the preparation of a dermatological composition, containing a physiologically acceptable medium, which is intended to prevent and/or combat skin irritation and/or dry patches and/or erythemas and/or pruritus in the skin.

2. Use according to the preceding claim, **characterized in that** an inhibitor of at least one excitatory amino acid is chosen from ketamine, memantine, (±)-2-amino-3-phosphonopropionic acid, (±)-2-amino-4-phosphonobutyric acid, (±)-2-amino-5-phosphonopentanoic acid, D-8-glutamylaminomethanesulfonic acid, 1,2,3,6,7,8-hexahydro-3-(hydroxyimino)-N,N,7-trimethyl-2-oxobenzo[2,1-b:3,4-c']dipyrrole-5-sulfonamide hydrochloride (referred to more commonly as: NS 257, HCl), cyclothiazide, aniracetam and riluzole.

3. Use according to Claim 2, **characterized in that** the inhibitor of at least one excitatory amino acid is chosen from aniracetam and ketamine.

4. Use according to one of the preceding claims, **characterized in that** the inhibitor of at least one excitatory amino acid is used in an amount ranging from 0.000001 to 5% by weight relative to the total weight of the composition.

5. Use according to one of the preceding claims, **characterized in that** the inhibitor of at least one excitatory amino acid is used in an amount ranging from 0.0001 to 1% by weight relative to the total weight of the composition.

6. Use according to one of the preceding claims, **characterized in that** the medium is an aqueous or aqueous-alcoholic solution, a water-in-oil emulsion, an oil-in-water emulsion, a microemulsion, an aqueous gel, an anhydrous gel, a serum or a vesicle dispersion.

7. Use according to any one of the preceding claims, **characterized in that** the composition contains at least one agent chosen from antibacterial agents, antiparasitic agents, antifungal agents, antiinflammatory agents, antipruriginous agents, anaesthetics, antiviral agents, keratolytic agents, anti-free-radical agents, antiseborrhoeic agents, antidandruff agents, antiacne agents and/or agents which modify skin differentiation and/or proliferation and/or pigmentation.

8. Cosmetic or dermatological composition containing a physiologically acceptable medium and at least one active agent with an irritant side effect, **characterized in that** it also contains at least one inhibitor of at least one excitatory amino acid.

9. Composition according to Claim 8, **characterized in that** the inhibitor of at least one excitatory amino acid is chosen from ketamine, memantine, (±)-2-amino-3-phosphonopropionic acid, (±)-2-amino-4-phosphonobutyric acid, (±)-2-amino-5-phosphonopentanoic acid, D-8-glutamylaminomethanesulfonic acid, 1,2,3,6,7,8-hexahydro-3-(hydroxyimino)-N,N,7-trimethyl-2-oxobenzo[2,1-b:3,4-c']dipyrrole-5-sulfonamide hydrochloride (referred to more commonly as: NS 257, HCl), cyclothiazide, aniracetam and riluzole.

10. Composition according to either of Claims 8 and 9, **characterized in that** the inhibitor of at least one excitatory amino acid is used in an amount ranging from 0.000001 to 5% of the total weight of the composition.

11. Composition according to one of Claims 8 to 10, **characterized in that** the inhibitor of at least one excitatory amino acid is used in an amount ranging from 0.0001 to 1% of the total weight of the composition.

12. Composition according to one of Claims 8 to 11, **characterized in that** the active agent with an irritant side effect is chosen from α-hydroxy acids, β-hydroxy acids, α-keto acids, β-keto acids, retinoids, anthralins, anthranoids, peroxides, lithium salts, antimetabolites, vitamin D and its derivatives.

13. Composition according to one of Claims 8 to 12, **characterized in that** it also contains at least one agent chosen from antibacterial agents, antiparasitic agents, antifungal agents, antiinflammatory agents, antipruriginous agents, anaesthetics, antiviral agents, keratolytic agents, anti-free-radical agents, antiseborrhoeic agents, antidandruff agents, antiacne agents and/or agents which modify skin differentiation and/or proliferation and/or pigmentation.

## Patentansprüche

1. Verwendung mindestens eines Inhibitors mindestens einer exzitatorischen Aminosäure für die Herstellung einer dermatologischen Zusammensetzung, die ein physiologisch akzeptables Medium enthält und die dazu vorgesehen ist, Hautreizungen und/oder Flechten und/oder Erythemen und/oder Juckreiz der Haut vorzubeugen und/oder diese zu bekämpfen.

2. Verwendung nach dem vorhergehenden Anspruch, **dadurch gekennzeichnet, dass** der Inhibitor mindestens einer exzitatorischen Aminosäure unter Ketamin, Memantin, (±)-2-Amino-3-phosphonopropionsäure, (±)-2-Amino-4-phosphonobuttersäure, (±)-2-Amino-5-phosphonopentansäure, D-8-Glutamylaminomethansulfonsäure, 1,2,3,6,7,8-Hexahydro-3-(hydroxyimino)-N,N,7-trimethyl-2-oxo-benzo[2,1-b:3,4-c']dipyrrol-5-sulfonamid-Hydrochlorid (das gewöhnlich als NS 257, HCl bezeichnet wird), Cyclothiazid, Aniracetam und Riluzol ausgewählt ist.

3. Verwendung nach Anspruch 2, **dadurch gekennzeichnet, dass** der Inhibitor mindestens einer exzitatorischen Aminosäure unter Aniracetam und Ketamin ausgewählt ist.

4. Verwendung nach einem der vorhergehenden Ansprüche, **dadurch gekennzeichnet, dass** der Inhibitor mindestens einer exzitatorischen Aminosäure in einem Mengenanteil von 0,000001 bis 5 Gew.-%, bezogen auf das Gesamtgewicht der Zusammensetzung, verwendet wird.

5. Verwendung nach einem der vorhergehenden Ansprüche, **dadurch gekennzeichnet, dass** der Inhibitor mindestens einer exzitatorischen Aminosäure in einem Mengenanteil von 0,0001 bis 1 Gew.-%, bezogen auf das Gesamtgewicht der Zusammensetzung, verwendet wird.

6. Verwendung nach einem der vorhergehenden Ansprüche, **dadurch gekennzeichnet, dass** es sich bei dem Medium um eine wässrige oder wässrig-alkoholische Lösung, eine Wasser-in-Öl-Emulsion, eine Öl-in-Wasser-Emulsion, eine Mikroemulsion, ein wässriges Gel, ein wasserfreies Gel, ein Serum, eine Vesikeldispersion handelt.

7. Verwendung nach einem der vorhergehenden Ansprüche, **dadurch gekennzeichnet, dass** die Zusammensetzung mindestens einen Stoff enthält, der unter den antibakteriellen Wirkstoffen, antiparasitären Wirkstoffen, antimykotischen Wirkstoffen, entzündungshemmenden Wirkstoffen, juckreizstillenden Wirkstoffen, anästhesierenden Wirkstoffen, antiviralen Wirkstoffen, Keratolytika, Radikalfängern für freie Radikale, antiseborrhoischen Wirkstoffen, Antischuppenmitteln, Wirkstoffen gegen Akne und/oder Wirkstoffen, die die Differenzierung und/oder die Proliferation und/ oder die Pigmentierung der Haut verändern, ausgewählt ist.

8. Kosmetische oder dermatologische Zusammensetzung, die ein physiologisch akzeptables Medium und mindestens einen Wirkstoff mit reizender Nebenwirkung enthält, **dadurch gekennzeichnet, dass** sie ferner mindestens einen Inhibitor mindestens einer exzitatorischen Aminosäure enthält.

9. Zusammensetzung nach Anspruch 8, **dadurch gekennzeichnet, dass** der Inhibitor mindestens einer exzitatorischen Aminosäure unter Ketamin, Memantin, (±)-2-Amino-3-phosphonopropionsäure, (±)-2-Amino-4-phosphonobuttersäure, (±)-2-Amino-5-phosphonopentansäure, D-8-Glutamylaminomethansulfonsäure, 1,2,3,6,7,8-Hexahydro-3-(hydroxyimino)-N,N,7-trimethyl-2-oxobenzo[2,1-b:3,4-c']dipyrrol-5-sulfonamid-Hydrochlorid (das gewöhnlich als NS 257, HCl bezeichnet wird), Cyclothiazid, Aniracetam und Riluzol ausgewählt ist.

10. Zusammensetzung nach einem der Ansprüche 8 oder 9, **dadurch gekennzeichnet, dass** der Inhibitor mindestens einer exzitatorischen Aminosäure in einer Menge von 0,000001 bis 5 % des Gesamtgewichts der Zusammensetzung verwendet wird.

11. Zusammensetzung nach einem der Ansprüche 8 bis 10, **dadurch gekennzeichnet, dass** der Inhibitor mindestens einer exzitatorischen Aminosäure in einer Menge von 0,0001 bis 1 % des Gesamtgewichts der Zusammensetzung verwendet wird.

12. Zusammensetzung nach einem der Ansprüche 8 bis 11, **dadurch gekennzeichnet, dass** der Wirkstoff mit reizender Nebenwirkung unter den α-Hydroxysäuren, β-Hydroxysäuren, α-Ketosäuren, β-Ketosäuren, Retinoiden, Anthralinen, Anthranoiden, Peroxiden, Lithiumsalzen, Antimetaboliten, Vitamin D und seinen Derivaten ausgewählt ist.

13. Zusammensetzung nach einem der Ansprüche 8 bis 12, **dadurch gekennzeichnet, dass** sie ferner mindestens einen Stoff enthält, der unter den antibakteriellen Wirkstoffen, antiparasitären Wirkstoffen, antimykotischen Wirkstoffen, entzündungshemmenden Wirkstoffen, juckreizstillenden Wirkstoffen, anästhesierenden Wirkstoffen, antiviralen Wirkstoffen, Keratolytika, Radikalfängern für freie Radikale, antiseborrhoischen Wirkstoffen, Antischuppenmitteln, Wirkstoffen gegen Akne und/ oder Wirkstoffen, die die Differenzierung und/oder die Proliferation und/oder die Pigmentierung der Haut verändern, ausgewählt ist.
